# EUROPÄISCHE PATENTANMELDUNG

(11) **EP 0 959 080 A1**
(43) Veröffentlichungstag der Anmeldung: **24.11.1999**
(21) Anmeldenummer: 99107085.5
(22) Anmeldetag: 12.04.1999
(51) Int. Cl.: C08F 4/04, C07D 237/26, C07D 487/04, C07D 231/54, C07D 491/02, C07D 271/12, C07F 7/08, C07C 271/60

(54) **Initiatoren für die radikalische Polymerisation**

(30) Priorität: 18.05.1998 US 80239
(71) Anmelder: BASF AKTIENGESELLSCHAFT, 67056 Ludwigshafen (DE)
(72) Erfinder: Königer, Rainer Dr., 67061 Ludwigshafen (DE); Schwalm, Reinhold Dr., 67157 Wachenheim (DE); Raether, Roman Benedikt Dr., 67061 Ludwigshafen (DE)

(57) **Zusammenfassung**

Initiatoren für die radikalische Polymerisation, enthaltend als Strukturmerkmal das Diels-Alder-Addukt einer Azogruppe (-N=N-) an eine konjugierte Doppelbindung (Dien).

## Beschreibung

Die Erfindung betrifft Initiatoren für die radikalische Polymerisation, enthaltend als Strukturmerkmal das Diels-Alder-Addukt einer Azogruppe (-N=N-) an eine konjugierte Doppelbindung (Dien).

Initiatoren für die radikalische Polymerisation sind Verbindungen, die in Radikale zerfallen und so die radikalische Polymerisation initiieren. Die Zerfallsgeschwindigkeit ist im allgemeinen temperaturabhängig und kann durch die Arrhenius-Gleichung beschrieben werden. Der Zerfall in Radikale beginnt demnach schon bei tieferen Temperaturen und wird durch Temperaturerhöhung beschleunigt.

Für manche Anwendungsgebiete, z.B. für die Härtung von Pulverlacken sind Initiatoren gewünscht, die erst bei hohen Temperaturen wirksam werden. Pulverlacke müssen nach dem Auftragen auf die Substratoberfläche im allgemeinen bis zur Fließfähigkeit erhitzt werden, um eine glatte und ebene Lackschicht zu bekommen. Eine gleichzeitig einsetzende Härtung (radikalisch Vernetzung) durch Zerfall der Initiatoren in Radikale führt dabei zu Unebenheiten bzw. Defekten in der Lackoberfläche. Die Härtung soll vielmehr erst einsetzen nachdem durch Aufschmelzen des Polymerpulvers eine glatte Lackschicht gebildet wurde.

Dazu werden Initiatoren benötigt, die erst bei hohen Temperaturen in Radikale zerfallen. Die Temperaturabhängigkeit der Zerfallsgeschwindigkeit soll dabei möglichst gering sein, das heißt kein Zerfall unterhalb einer bestimmten Temperatur möglichst vollständiger Zerfall oberhalb dieser Temperatur (nicht-Arrhenius-Verhalten).

Aufgabe der vorliegenden Erfindung war, entsprechende Initiatoren zur Verfügung zu stellen.

Demgemäß wurden die eingangs definierten Initiatoren gefunden.

Die erfindungsgemäßen Initiatoren enthalten als Strukturmerkmal das Diels-Alder-Addukt einer Azogruppe, das heißt einer -N=N- Gruppe, an ein Dien.

Bei einer Diels-Alder-Reaktion handelt es sich um eine 1,4-Cycloaddition einer C,C-Doppelbindung (stattdessen hier eine Azogruppe) an eine konjugierte Doppelbindung (Dien).

Entsprechend können die erfindungsgemäßen Initiatoren erhalten werden durch 1,4-Cycloaddition der Azogruppe einer Azoverbindung (Dienophil) an ein Dien.

Bei der Azoverbindung handelt es sich um eine Verbindung, welche bereits ein Initiator für die radikalische Polymerisation ist und oberhalb bestimmter Temperaturen in Radikale zerfällt (Azoinitiator). Weiterhin sollte die Azoverbindung eine Diels-Alder Reaktion eingehen können. Geeignete Azoverbindungen sind z.B. solche der Formel 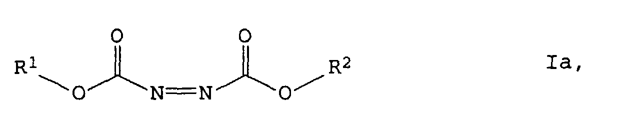 worin R¹ und R² unabhängig voneinander für organische Reste stehen.

Bevorzugt handelt es sich bei den Resten R¹ und R² um organische Reste, welche bis zu 30, insbesondere bis zu 20 C-Atome und gegebenenfalls noch Heteroatome wie N, O, S enthalten.

Besonders bevorzugt handelt es sich bei R¹ und R² unabhängig voneinander um eine C₁-C₂₀-Alkylgruppe, eine C₅-C₂₀-Arylgruppe oder Cycloalkylgruppe, eine C₆- bis C₂₀-Alkaryl- oder Aralkylgruppe. Insbesondere handelt es sich um eine C₁-C₈ Alkylgruppe. Die Alkylgruppen können linear oder verzweigt sein.

Eine weitere zur Diels-Alder-Reaktion befähigte Azoverbindung ist ein Imid der Formel

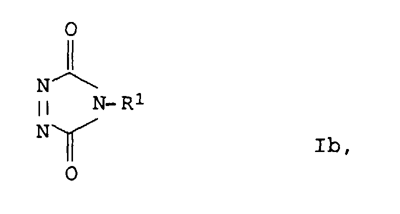

R¹ hat die oben angegebene Bedeutung.

Die initiierende Wirkung der Azodicarbonsäureester ist in der Literatur beschrieben (T. Schmelzer und J.Springer; Eur.Polym.J.23(3), pp. 243-248 (1987)).

Als Diene, welche mit den Azoverbindungen das Diels-Alder-Addukt bilden, kommen z.B. Butadien, Cyclopentadien, Cyclohexadien, oder deren Derivate, in denen ein oder mehrere der Wasserstoffatome durch organische Reste substituiert sind in Betracht.

Wesentlich ist lediglich, daß eine konjugierte Doppelbindung vorhanden ist, welche zu einer Diels-Alder-Reaktion befähigt ist.

Die Durchführung der Diels-Alder-Reaktion ist bekannt.

Zur Herstellung der erfindungsgemäßen Initiatoren können die Azoverbindungen mit dem Dien z.B. bei Temperaturen von 0°C bis 100°C in einem Lösemittel umgesetzt werden.

Bei der Umsetzung der Azoverbindungen der Formel I z.B. mit Cyclopentadien wird folgendes Diels-Alder-Addukt erhalten:

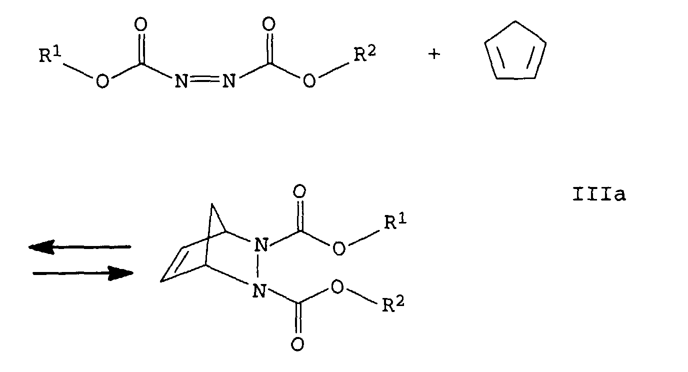

Auch beim erfindungsgemäßen Initiator ist die ursprüngliche Azoverbindung die Verbindung, die in Radikale zerfällt und so initiierend wirkt. Nur muß diese Azoverbindung zunächst durch eine Umkehr der Diels-Alder-Reaktion (Retro-Diels-Alder-Reaktion) aus den Diels-Alder-Addukt freigesetzt werden.

Die Temperatur, welche für die Retro-Diels-Alder-Reaktion benötigt wird, hängt von der Wahl der Azoverbindung mit der Dienkomponenten ab. Im allgemeinen liegen die Temperaturen oberhalb 100°C,insbesondere zwischen 150 und 250°C. Diese Temperaturen liegen im allgemeinen oberhalb der Zerfallstemperatur der Azoverbindungen. Die Azoverbindung wird bei Temperaturerhöhung erst nach erfolgter Retro-Diels-Alder-Reaktion freigesetzt,so daß die Zerfallstemperatur der erfindungsgemäßen Initiatoren in Radikale gegenüber den ursprünglichen Azoverbindungen erhöht ist.

Auch die Retro-Diels-Alder-Reaktion folgt dem Arrhenius-Gesetz, d.h. sie zeigt eine gewisse Temperaturabhängigkeit. Eine weitere Verbesserung dieser Erfindung kann erreicht werden, wenn diese Temperaturabhängigkeit noch weiter vermindert wird.

Aus der Literatur Fréchet, J.M.J; Eichler, E, HO, H; Willson C.G. Polymer 1996, 24, 995-1000 ist bekannt, daß Phenyl-tert.-Butylcarbonat unter thermischer Belastung schlagartig in iso-Buten zerfällt:

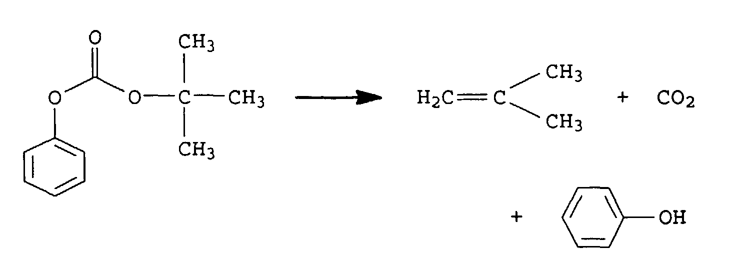

Der Zerfall ist autokatalytisch und folgt nicht dem Arrhenius-Gesetz.

Z.B. ergibt sich bei entsprechender Addition von Substituenten an das Diels-Alder-Addukt der Formel IIIa die Verbindung 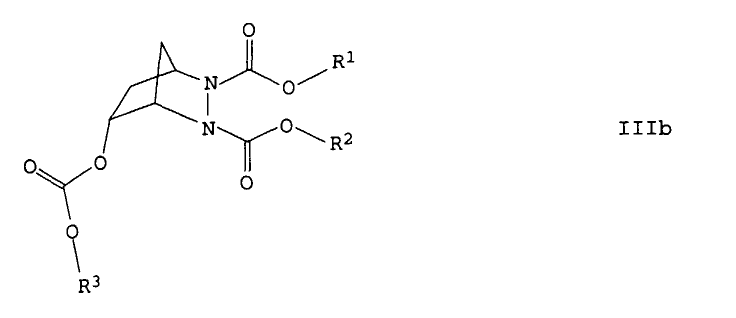 worin R³ für eine Phenylgruppe, welche gegebenenfalls noch weitere Substituenten trägt, steht.

Die Verbindung der Formel IIIb zerfällt bei Temperaturerhöhung unter Abspaltung von CO₂ und Phenol autokatalytisch in das Diels-Alder-Addukt der Formel IIIa und dies dann weiter in einen Retro-Diels-Alder-Reaktor in die als Initiator wirksame Azoverbindung.

Auf diese Weise wird erreicht, daß eine Temperaturabhängigkeit des Zerfalls des Diels-Alder Addukts unterbunden wird und der im Diels Alder Addukt enthaltende Initiator in vollem Umfang erst oberhalb der Temperatur des autokatalytischen Zerfalls wirksam wird.

Diels Alder Addukte, die einen entsprechenden autokatalytischen Zerfall unterliegen sind solche, die durch mindestens eine Gruppe der Formeln 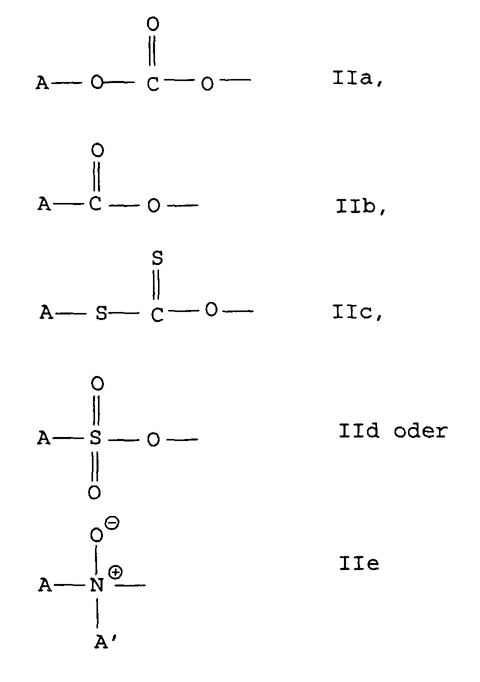 substituiert sind.

A und A' stehen in obigen Formeln unabhängig voneinander für organische Reste mit 1 bis 30 C-Atomen, vorzugsweise 2 bis 20 C-Atomen.

Insbesondere handelt es sich um einen Rest mit einem aromatischen Ringsystem. Besonders bevorzugt handelt es sich um eine Phenylgruppe, welche gegebenenfalls noch weitere Substituenten, z.B. C₁-C₈ Alkylgruppen tragen kann. Ganz besonders bevorzugt ist eine Phenylgruppe.

Bevorzugte Substituenten sind Gruppen der Formel IIa oder IId, insbesondere Phenylcarbonat oder Phenylsulfonat.

Das Diels Alder Addukt enthält vorzugsweise ein oder zwei, besonders bevorzugt einen Substituenten der Formel IIa bis IIe.

Die Phenylgruppe des Phenylcarbonats bzw. Phenylsulfonats kann weitere Substituenten enthalten, insbesondere z.B. C₁-C₈ Alkylgruppen. Die autokatalytische Zerfallstemperatur kann dadurch variiert werden.

Die obigen Gruppen der Formeln IIa) bis IIe), insbesondere die Phenylcarbonat bzw. Phenylsulfonatgruppe können an das Diels-Alder Addukt z.B. durch übliche Additionsreaktion an die bei der Diels-Alder-Reaktion erhaltenen Doppelbindungen gebunden werden. Geeignet sind insbesondere auch Veresterungen der von IIa bis IIe abgeleiteten Säuren, Säureanhydriden oder Säurechloriden mit Hydroxygruppen enthaltenden Diels-Alder Addukten. Besonders einfach gelingt die Anbindung der Phenylcarbonat- oder Phenylsulfonatgruppe bzw. deren Derivate durch Umsetzung von 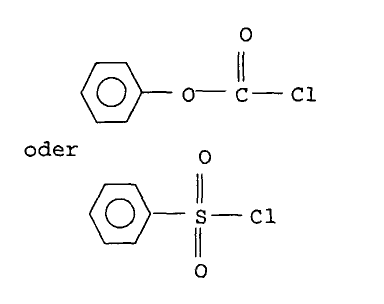 mit Hydroxygruppen enthaltenden Diels-Alder Addukten.

Die erfindungsgemäßen Initiatoren eignen sich als Initiatoren für die radikalischen Polymerisation. Es kann sich dabei um die Polymerisation von niedermolekularen Monomeren, z.B. Acrylaten, Vinylaromaten, Vinylestern, Vinylethern etc. handeln. In Betracht kommt auch die Vernetzung von Oligomeren und Polymeren durch radikalische Polymerisation von radikalisch polymerisierbaren, ethylenisch ungesättigten Gruppen, welche in dem Polymer bzw. Oligomer enthalten sind. Zum Beispiel können Polymere oder Oligomere Epoxidgruppen enthalten, welche mit Acrylsäure oder Hydroxyalkylacrylaten umgesetzt werden, und so der gewünschte Gehalt an Doppelbindungen eingestellt wird. Von besonderem Interesse sind die erfindungsgemäßen Initiatoren für Polymerpulver, welche durch radikalische Polymerisation vernetzt bzw. gehärtet werden.

Der Gehalt der erfindungsgemäßen Initiatoren beträgt im allgemeinen 0,05 bis 10 Gew.-Teile, insbesondere 0,1 bis 5 Gew.-Teile, bezogen auf 100 Gew.-Teile der insgesamt vorhandenen radikalisch polymerisierbaren Monomeren und/oder radikalisch vernetzbaren Oligomere oder Polymere.

### Beispiele

### 1. Herstellung von Initiatoren

### 1.1. Diels-Alder-Addukte

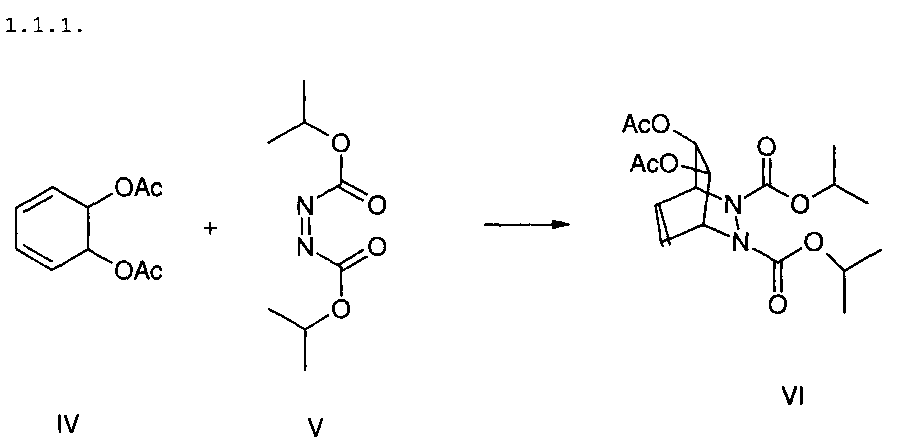

7,7 g IV und 7,9 g V werden in 400 ml Cyclohexan gelöst und 20 Stunden bei 50 °C mit UV-Licht bestrahlt. Nach der Reaktion wird das Lösungsmittel abdestilliert und das Rohprodukt chromatographisch gereinigt. Ausbeute: 6,2 g VI.

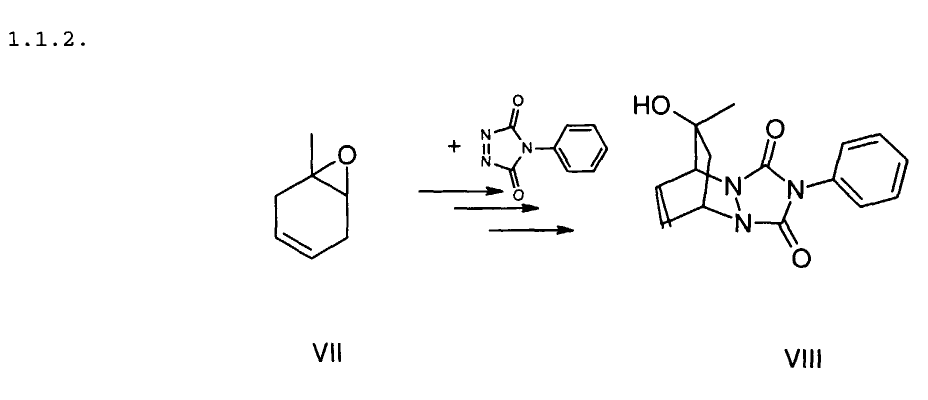

2,2 g VII werden in Diethylether gelöst und bei 0 °C zu einer 1,4 molaren Lösung von Methyllithium in Diethylether getropft, eine Stunde bei Raumtemperatur gerührt und 10 ml einer 5%igen Natriumhydroxid-Lösung zugegeben. Die organische Phase wird mit gesättigter Kochsalziösung gewaschen und 4-Phenylurazol zugesetzt.

Das Lösungsmittel wird abdestilliert und das Zwischenprodukt chromatographisch gereinigt. Man erhält 1,6 g eines weißen Feststoffes.
3,27 g Chromtrioxid werden in 80 ml Methylenchlorid gelöst und 5,2 g Pyridin unter Eiskühlung zugesetzt. Zu dieser Lösung wird der oben erwähnte weiße Feststoff, gelöst in 10 ml Methylenchlorid, bei 0 °C zugetropft. Der Ansatz wird 1 Stunde bei Raumtemperatur gerührt, und dekantiert. Die Lösung wird gewaschen, getrocknet und eingeengt. Das Rohprodukt wird chromatographisch und durch Umkristallisation gereinigt. Ausbeute: 165 mg VIII (weiße Kristalle).

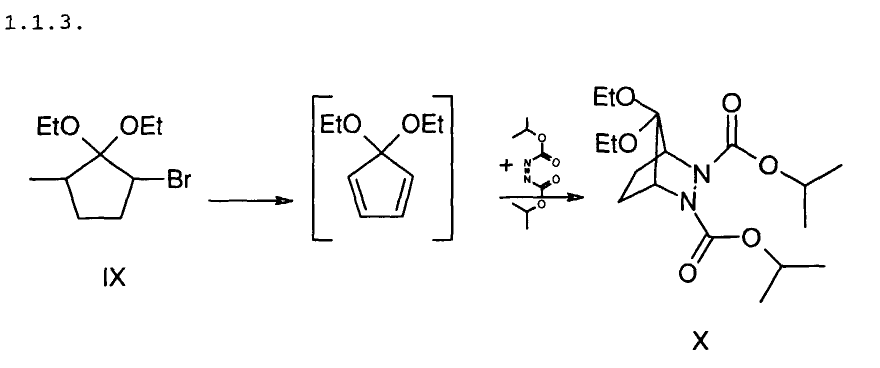

8,9 g IX werden in 25 ml Dimethylsulfoxid gelöst und bei 17 °C zu einer Lösung von 12,84 g Kalium-t-butylat in 75 ml Dimethylsulfoxid getropft. 100 ml kaltes Pentan werden zugegeben und die Mischung in 100 ml Eis gegossen. Die organische Phase wird abgetrennt, bei 0 °C gehalten und innerhalb einer Stunde zu einer Lösung von 23,24 g Diisopropylazodicarboxylat in 30 ml Diethylether getropft. Die Lösung wird anschließend 2,5 h zum Sieden erhitzt.
Die flüchtigen Bestandteile werden abdestilliert und das erhaltene gelbe Öl in n-Hexan gelöst und im Kühlschrank aufbewahrt. Über Nacht fallen 1,18 g X (weiße Kristalle) aus.

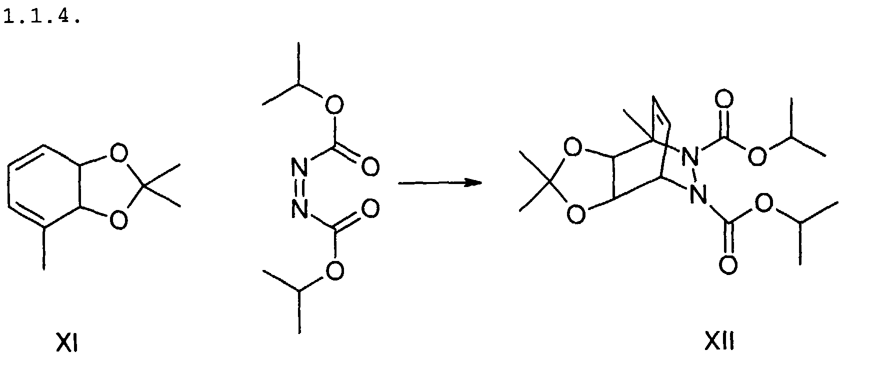

200 mg XI und 200 mg Diisopropylazodicarboxylat werden in Benzol gelöst und die Lösung 24 h zum Sieden erhitzt. Das Lösungsmittel wird abdestilliert und das Rohprodukt chromatographisch gereinigt. Ausbeute: 220 mg XII (farbloses Öl).

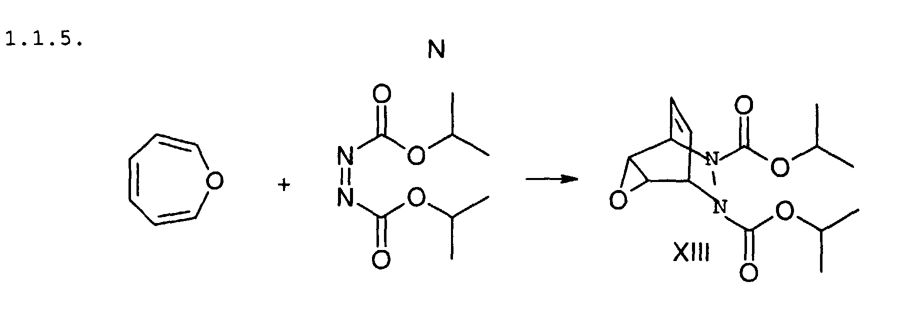

577 mg Oxepin werden in 10 ml Chloroform gelöst und 1210 mg Diisopropylazodicarboxylat, gelöst in 4 ml Chloroform, innerhalb von 30 min zugegeben. Der Ansatz wird 21 h bei Raumtemperatur gerührt. Die flüchtigen Bestandteile werden abdestilliert und der Rückstand umkristallisiert. Ausbeute: 1466 mg XIII (weiße Kristalle).

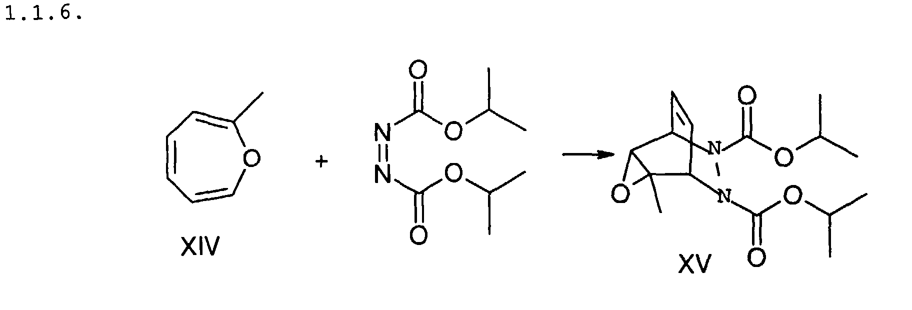

560 mg XIV werden in 6 ml Chloroform gelöst und 1070 mg Diisopropyldiazocarboxylat, gelöst in 2 ml Chloroform, zugegeben. Der Ansatz wird 24 h bei Raumtemperatur gerührt. Die flüchtigen Bestandteile werden abdestilliert und der Rückstand chromatographisch gereinigt. Ausbeute: 710 mg XV (farbloses Öl) 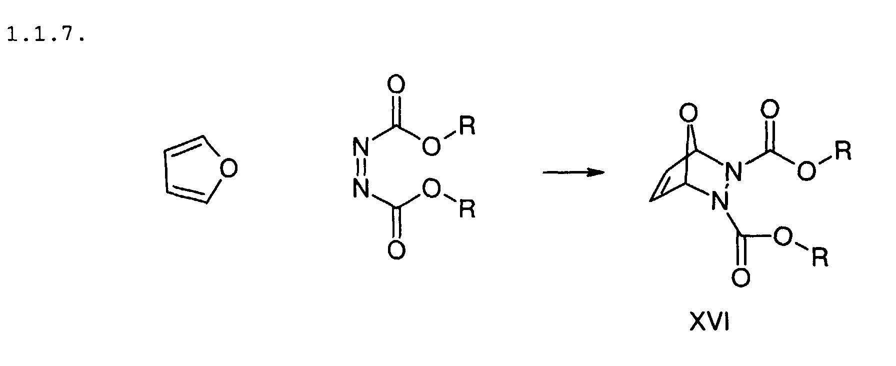 R = Ethyl, 2-Propyl

13,7 mmol des entsprechenden Dialkylazodicarboxylates werden in 10 ml Furan gelöst und 20 h bei Raumtemperatur gerührt. Nach Abdestillieren der flüchtigen Bestandteile bleibt das Produkt XVI (Öl) zurück.

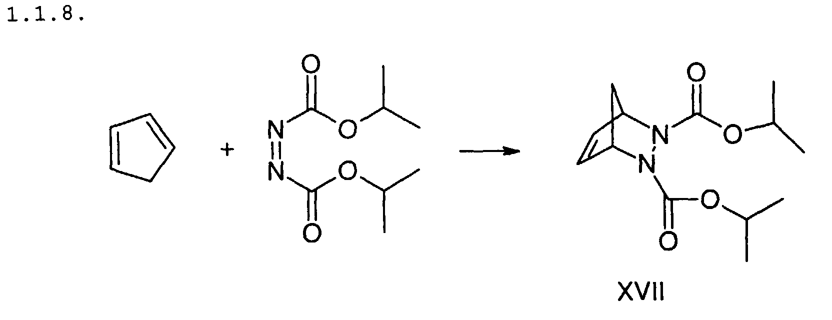

Zu 3,22 g eiskaltem Diisopropylazodicarboxylat wird Cyclopentadien getropft bis sich die Reaktionsiösung entfärbt hat. Die flüchtigen Bestandteile werden abdestilliert und der Rückstand chromatographisch gereinigt. Man erhält XVII als farbloses Öl.

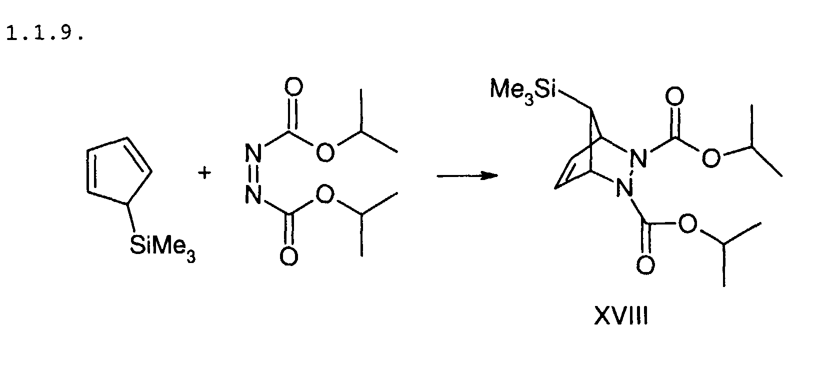

7,8 ml Trimethylsilylcyclopentadien und 6,71 g Diisopropylazodicarboxylat werden in Tetrahydrofuran gelöst und 18 h bei Rauintemperatur gerührt. Die flüchtigen Bestandteile werden abdestilliert und der Rückstand chromatographisch gereinigt. Ausbeute: 3,51 g XVIII (farbloses Öl).

### 1.2. Derivatisierung der beschriebenen Diels-Alder-Addukte

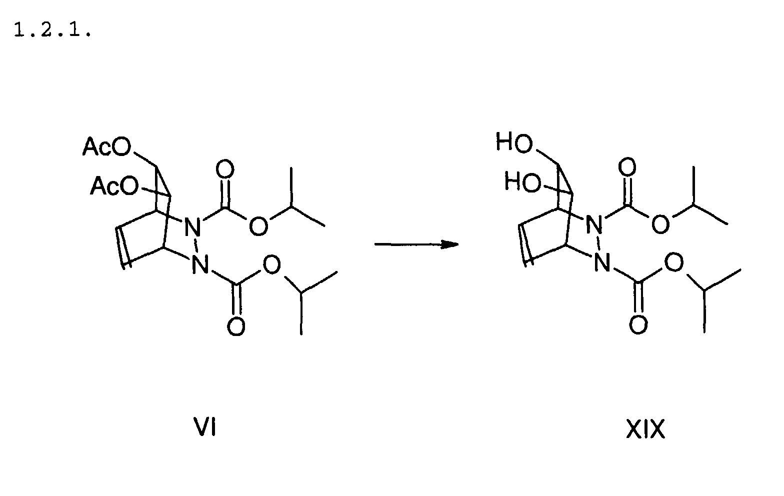

220 mg VI werden in 10 ml Diethylether gelöst und 51 mg Methanol sowie 35 mg Lithiumborhydrid zugegeben. Der Ansatz wird 2,5 h zum Sieden erhitzt, neutralisiert und extrahiert. Die organische Phase wird eingeengt und das erhaltene Öl chromatographisch gereinigt. Ausbeute: 36 mg XIX (farbloses Öl).

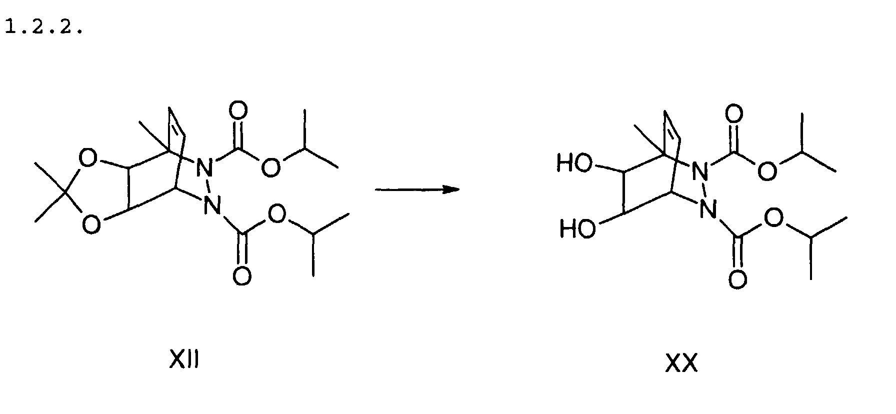

220 mg XII werden in 10 ml Methanol gelöst und eine Spatelspitze Amberlyst® 15 zugesetzt. Der Ansatz wird 24 h auf 70 °C erwärmt. Man erhält XX.

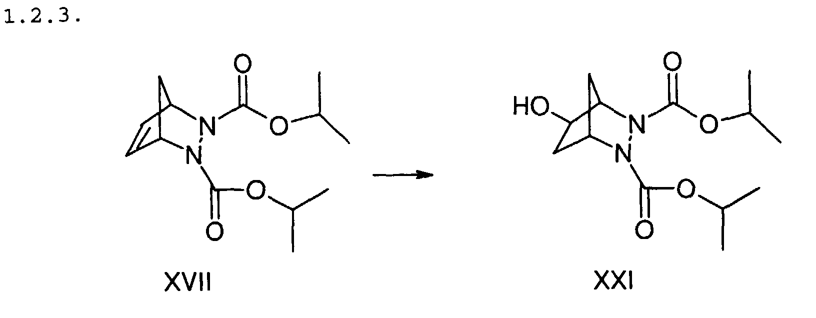

4,07 g XVII werden in 12 ml Tetrahydrofuran gelöst, auf 0 °C gekühlt, und 9,4 ml einer 1M Boran-Lösung zugegeben. Die Lösung wird 1 h unter Kühlung und 2 h bei Raumtemperatur gerührt. 2,8 ml 3M Natronlauge und 2 ml 30% Wasserstoffperoxyd werden zugesetzt und die Lösung 30 min bei 0 °C und 1 h bei Raumtemperatur gerührt. Die Lösung wird in Essigsäureethylester aufgenommen und mit gesättigter Kochsalzlösung gewaschen. Die organische Phase wird eingeengt und das Rohprodukt chromatographisch gereinigt. Ausbeute: 2,87 g XXI (farbloses Öl, kristallisiert nach einigen Tagen bei Raumtemperatur).

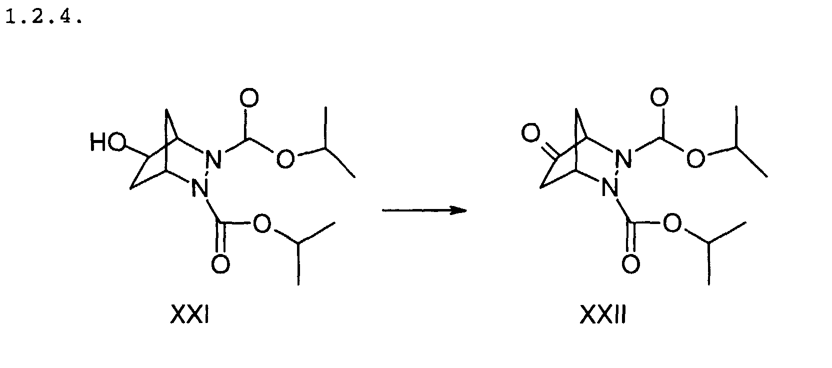

2,173 g XXI werden in 20 ml Methylenchlorid gelöst und 4,436 g Pyridiniumdichromat, 8 g Molekularsieb sowie 0,8 ml Essigsäure zugegeben. Der Ansatz wird 24 h gerührt und das Filtrat eingeengt. Der Rückstand wird in Diethylether aufgenommen, gewaschen und eingeengt. Das Rohprodukt wird chromatographisch gereinigt. Ausbeute: 0,869 g XXII (farbloses Öl, kristallisiert nach einigen Tagen bei Raumtemperatur).

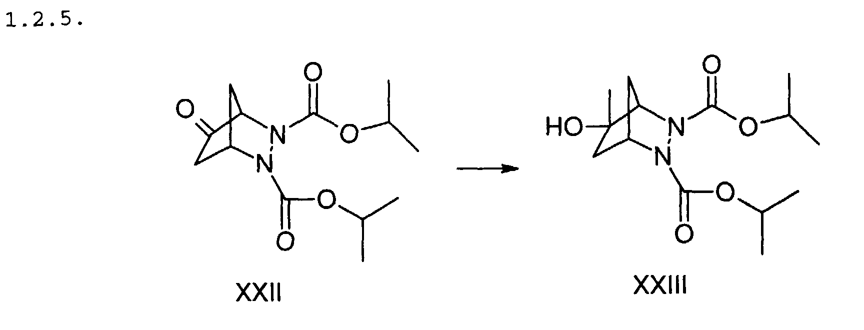

0,106 g XXII werden in 9 ml Tetrahydrofuran gelöst und 0,3 ml einer 3 M Methylmagnesiumchlorid - Lösung zugesetzt. Der Ansatz wird 2 h gerührt, mit Eis versetzt und die flüchtigen Bestandteile abdestilliert. Der Rückstand wird chromatographisch gereinigt. Ausbeute: 0,02 g XXIII (farbloses Öl).

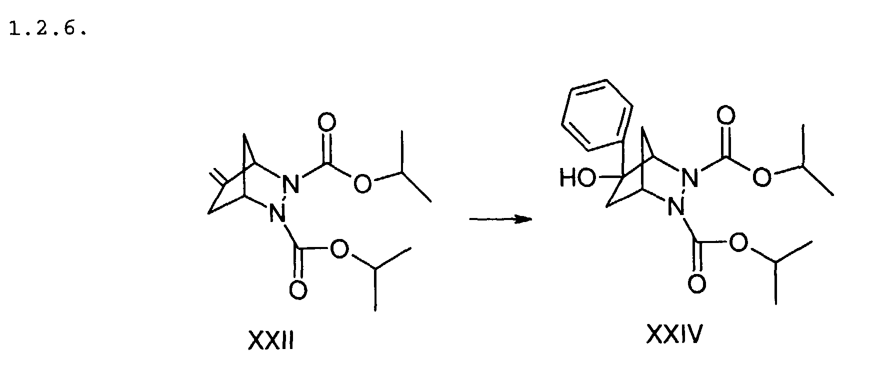

0,116 g XXII werden in 10 ml Tetrahydrofuran gelöst, auf -78 °C gekühlt und 0,465 ml einer 1M Phenylmagnesiumbromid - Lösung zugesetzt. Die Lösung wird 2 h bei -78 °C gerührt. Nach der Zugabe von Eis und Essigsäureethylester wird die organische Phase gewaschen und eingeengt. Das Rohprodukt wird chromatographisch gereinigt. Ausbeute: 0,082 g XXIV (gelbliches Öl).

### 1.3. Diels-Alder-Addukte mit Carbonat- bzw. Sulfonatgruppen

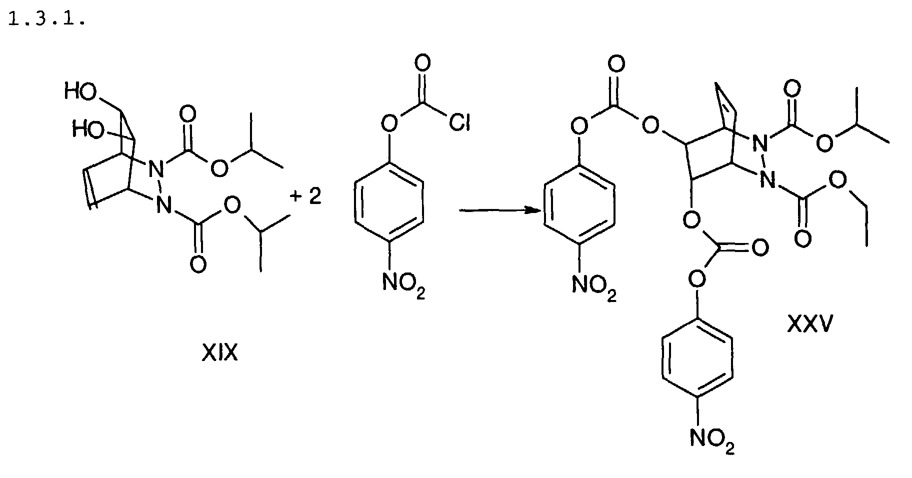

36 mg XIX werden in 2 ml Tetrahydrofuran gelöst und 0,03 ml Pyridin zugesetzt. 46,4 mg p-Nitrophenylchlorformiat werden bei 0 °C zugegeben und die Lösung bei Raumtemperatur 17 h gerührt. Nach Zugabe von je 1 ml Wasser und Diethylether wird die organische Phase abgetrennt, gewaschen und eingeengt. Das Rohprodukt wird chromatographisch gereinigt. Man erhält XXV.

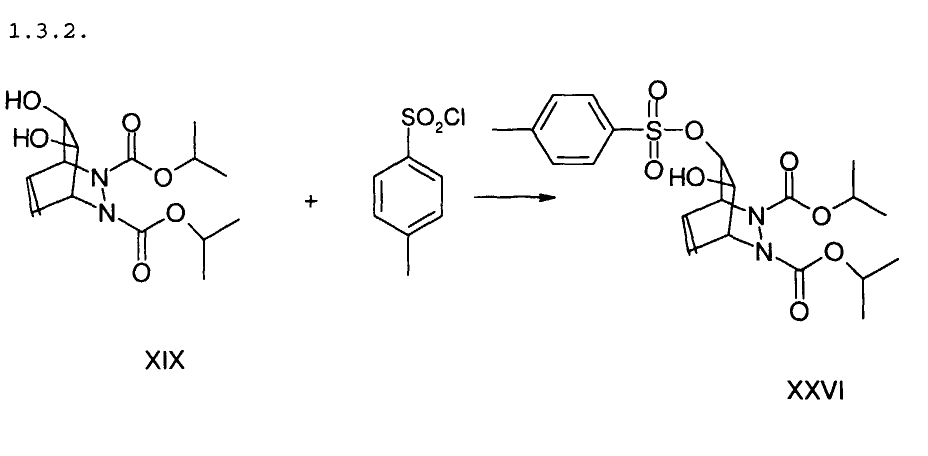

700 mg XIX werden in 10 ml Pyridin gelöst und 425,4 mg p-Toluolsulfonsäurechlorid bei 0°C zugegeben. Die Lösung wird 2 d im Kühlschrank aufbewahrt, dann in 40 ml salzsaures Eis gegossen. Die organische Phase wird eingeengt und das Produkt im Hochvakuum getrocknet. Ausbeute: 720 mg XXVI (weißer Feststoff).

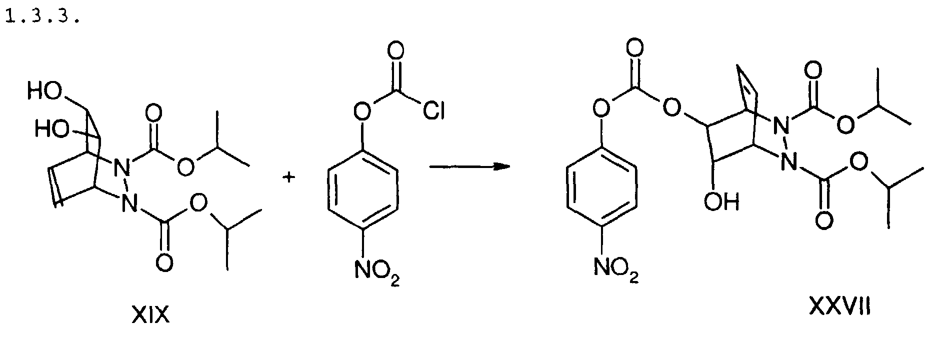

100 mg XIX werden in 20 ml Tetrahydrofuran gelöst und 0,042 ml Pyridin zugegeben. 63,5 mg p-Nitrophenylchlorformiat, gelöst in 6 ml Tetrahydrofuran, werden bei 0 °C innerhalb von 50 min zugetropft. Die Lösung wird 4,5 d im Kühlschrank aufbewahrt, dann je 3 ml Wasser und Diethylether zugegeben. Die organische Phase wird gewaschen und eingeengt. Das Rohprodukt wird chromatographisch gereinigt. Ausbeute: 70 mg XXVII.

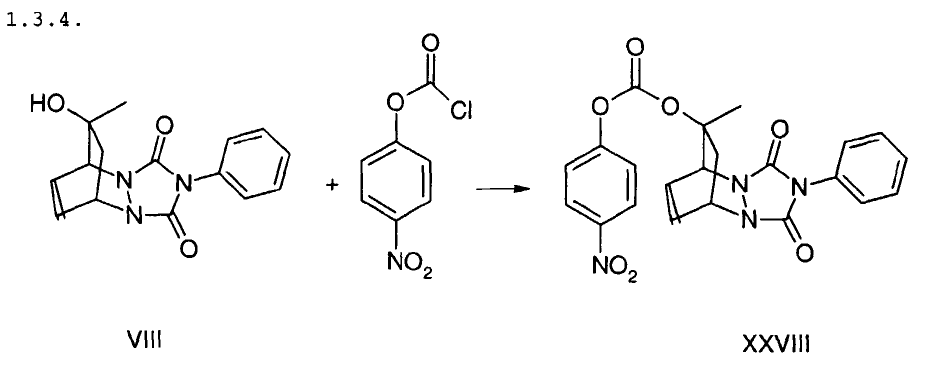

165 mg VIII werden in 7 ml Tetrahydrofuran gelöst und 0,1 ml Pyridin zugegeben. 121 mg p-Nitrophenylchlorformiat, gelöst in 3 ml Tetrahydrofuran, werden bei 0 °C zugesetzt und die Lösung bei 0°C 1,5 h, bei Raumtemperatur 17 h gerührt. Nach Zugabe von 10 ml Diethylether und 5 ml Wasser wird die organische Phase gewaschen und eingeengt. Das Rohprodukt wird durch Umkristallisation gereinigt. Ausbeute: 105 mg XXVIII (weißer Feststoff).

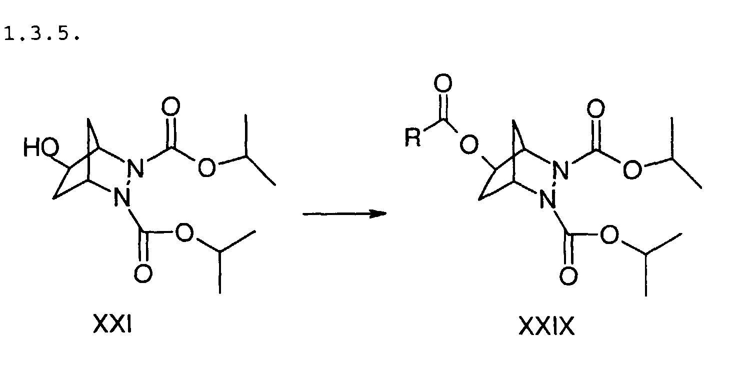

XXI wird im angegebenen Lösungsmittel gelöst und mit einem leichten Überschuß des entsprechenden Reaktanden versetzt. Man läßt bei Raumtemperatur rühren, destilliert die flüchtigen Bestandteile ab und reinigt das Rohprodukt chromatographisch.

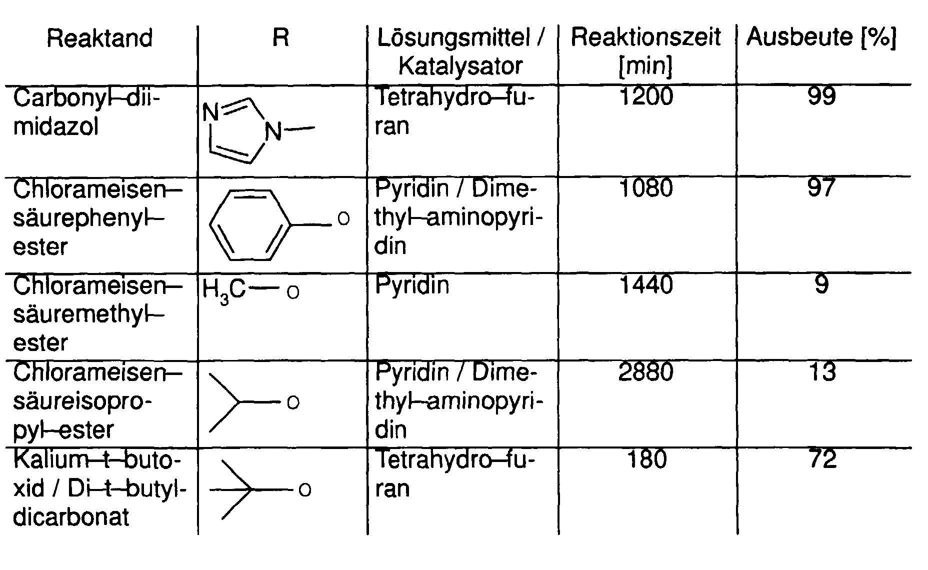

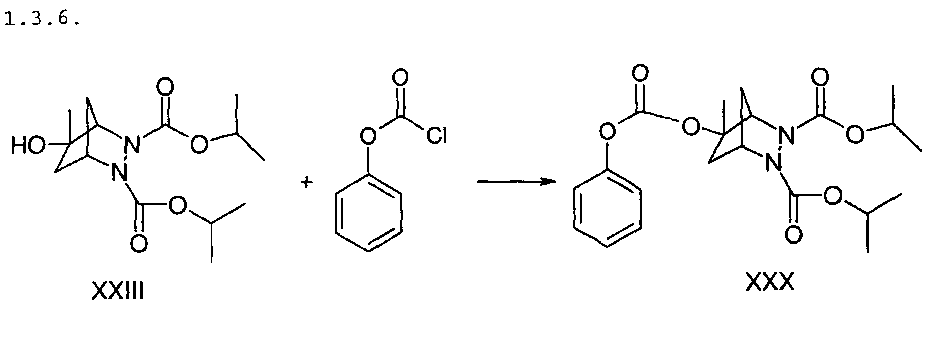

14,5 mg XXIII werden in 2,5 ml Methylenchlorid gelöst und nacheinander 13,1 mg Dimethylaminopyridin und 0,035 ml Chlorameisensäurephenylester zugesetzt. Der Ansatz wird 42 h bei Raumtemperatur gerührt. Die flüchtigen Bestandteile werden abdestilliert und das Rohprodukt chromatographisch gereinigt. Ausbeute: 15 mg XXX (farbloses Öl).

### 2. Polymerisation

### 2.1. Polymerisation ohne Initiator

Benzylmethacrylat wurde in einem Glasrohr unter Vakuum entgast, versiegelt und in einem Ölbad 1 Stunde lang auf 150 °C erhitzt. Danach war die Viskosität nur leicht angestiegen.

### 2.2. Polymerisation mit Initiator

### 2.2.1. Polymerisation mit XVII

### 2.2.1.1. Mit Benzylmethacrylat

Benzylmethacrylat und 5 Gew.-% XVII, bezogen auf Benzylmethacrylat, wurden in einem Glasrohr unter Vakuum entgast, versiegelt und in einem Ölbad 1 Stunde lang auf 150 °C erhitzt. Danach war der Ansatz nicht mehr fließfähig.

### 2.2.1.2. Mit n-Butylacrylat

n-Butylacrylat und 0,42 Gew.-% XVII, bezogen auf n-Butylacrylat, wurden in einem Glasrohr unter Vakuum entgast, versiegelt und in einem Ölbad 1 Stunde lang auf 145 °C erhitzt. Danach war der Ansatz hochviskos.

### 3. Zerfallstemperaturen

### 3.1. Zerfallstemperaturen von Alkylphenylcarbonaten

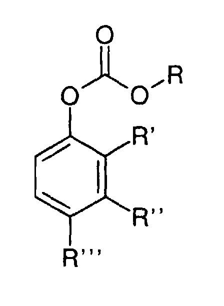

| Verbindung | R | R' | R'' | R''' | Zerfallstemperatur [°C] |
|---|---|---|---|---|---|
| 1 | t-Butyl | H | H | H | verfl. > 150 |
| 2 | t-Butyl | H | NO₂ | H | 150 |
| 3 | t-Butyl | Cl | H | Cl | verfl. > 170 |
| 4 | t-Butyl | H | H | NO₂ | 65 |
| 5 | 2-Propyl | H | H | NO₂ | verfl. > 250 |
| 6 | t-Butyl | H | H | Cl | 140 |
| 7 | t-Butyl | | perfluor | | verfl. > 80 |

### 3.2. Zerfallstemperaturen von Diels-Alder-Addukten mit Carbonat- bzw. Sulfo-natgruppierung

| Verbindung | Zerfallstemperatur [°C] |
|---|---|
| XXVI | 148 |
| XXVII | 170 |
| XXV | 195 |
| XXVIII | 245 |

## Patentansprüche

1. Initiatoren für die radikalische Polymerisation, enthaltend als Strukturmerkmal das Diels-Alder-Addukt einer Azogruppe (-N=N-) an eine konjugierte Doppelbindung (Dien).

2. Initiatoren gemäß Anspruch 1, wobei es sich bei den Initiatoren um ein Diels-Alder-Addukt einer Verbindung der Formel 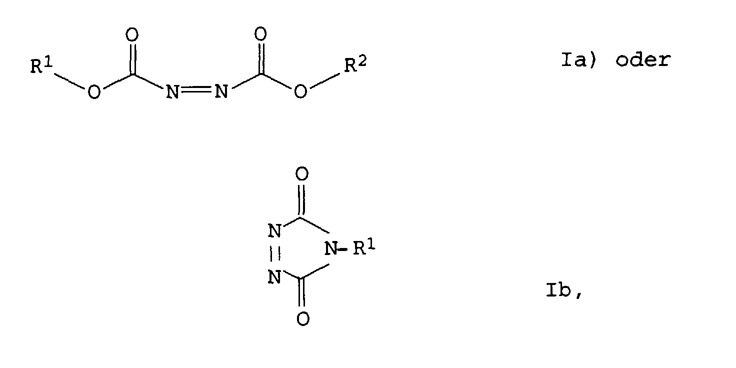 worin R¹ und R² und für organische Reste stehen, an ein Dien handelt.

3. Initiatoren gemäß einem der Ansprüche 1 oder 2, wobei es sich bei dem Dien um Butadien, Cyclopentadien, Cyclohexadien oder Derivate dieser Verbindungen handelt.

4. Initiatoren gemäß einem der Ansprüche 1 bis 3, wobei das Diels-Alder Addukt durch mindestens eine Gruppe der Formeln 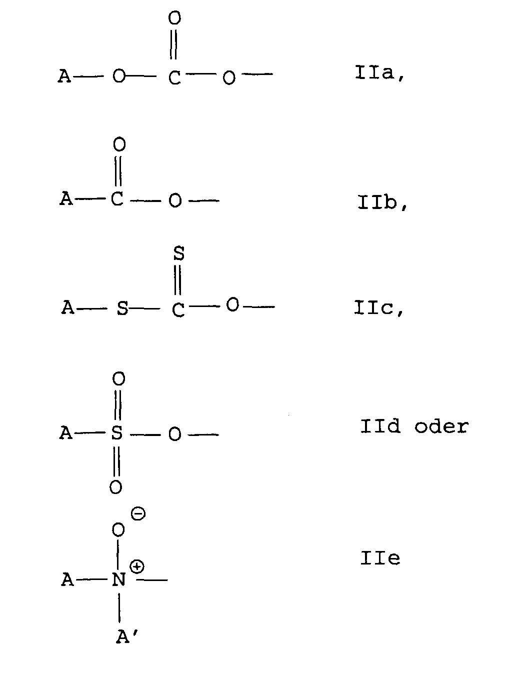 worin A und A' unabhängig voneinander für einen organischen Rest mit 1 bis 30 C-Atomen steht, substituiert ist.

5. Verwendung von Initiatoren gemäß einem der Ansprüche 1 bis 4 als Initiatoren für die radikalische Polymerisation.
